Europäisches Patentamt

European Patent Office

Office européen des brevets

(11) Publication number: 0 307 774
A2

# EUROPEAN PATENT APPLICATION

(21) Application number: 88114583.3

(22) Date of filing: 07.09.88

(51) Int. Cl.⁴: **A61K 37/54** , **A61K 37/12**

(30) Priority: 16.09.87 US 97035

(43) Date of publication of application:
22.03.89 Bulletin 89/12

(84) Designated Contracting States:
AT BE CH DE ES FR GB GR IT LI LU NL SE

(71) Applicant: W.R. Grace & Co.-Conn.
1114 Avenue of the Americas
New York New York 10022(US)

(72) Inventor: Parham, Marc Ellous
6 Ruben Duren Way
Bedford Massachusetts(US)
Inventor: Raina, Santosh
8 Hazel Road
Lexington Massachusetts(US)

(74) Representative: UEXKÜLL & STOLBERG
Patentanwälte
Beselerstrasse 4
D-2000 Hamburg 52(DE)

(54) Process for purifying plasminogen activator.

(57) Tissue plasminogen activator (t-PA) is isolated from a source material (i.e., conditioned cell culture media, tissue preparations or body fluids) by contacting with fibrin that has been pretreated by washing with t-PA elution buffer to remove elutable proteins, separating the fibrin-bound t-PA, then washing and eluting the t-PA. Salts and excess buffer are removed by dialysis to yield a highly purified t-PA product.

EP 0 307 774 A2

# PROCESS FOR PURIFYING TISSUE PLASMINOGEN ACTIVATOR

## BACKGROUND OF THE INVENTION

This invention relates generally to the purification of tissue plasminogen activator ("t-PA"). More specifically, a greatly simplified purification process has been discovered which yields purified t-PA in a single processing operation. The t-PA product of this invention is a highly purified composition which has not previously been isolated in that form in a single step process and with high yields.

The t-PAs or tissue plasminogen activators are believed to play a role in circulatory functions, including clot dissolution, by converting the proenzyme plasminogen into enzymatically active plasmin. Plasmin, a fibrolytic proteinase, acts to dissolve fibrin which is a major component of blood clots. The therapeutic use of t-PA to dissolve blood clots in heart attack victims has met with early and exciting success in clinical trials, and already has far surpassed the efficacy of the currently approved clot dissolving enzyme, streptokinase, as reported by Klausner, "Researchers Probe Second-Generation t-PA," BIO/TECHNOLOGY, Vol. 4, pp. 707-11 (1986). The specificity of t-PAs for the fibrin component of blood clots has the clinically important advantage of avoiding systemic degradation of blood proteins, such as fibrinogen, which would result in internal bleeding.

One current drawback to using t-PAs therapeutically is the difficulty in isolating them from tissue or cell culture medium, or from body fluids. Cells from tissues known to secrete t-PA may be cultured for the production and secretion of t-PA into the medium. This "conditioned medium" is collected and subjected to various purification processes. There are a variety of known t-PAs, produced by various tissues, e.g., uterine tissue, endothelial cells and the like. Previously known methods for purifying t-PA have been quite elaborate, requiring many steps. In addition, yields from conventional processes range only from about 20 to about 60%. Moreover, the t-PA product resulting from conventional processes may still contain protein contaminants.

U.S. 4,381,346 (Hussin et al.) discloses a method of isolating plasminogen activator from urine or kidney tissue culture medium in a procedure in which the plasminogen activator is bound to immobilized fibrin, eluted and then passed through a gel filtration column to separate the plasminogen activator from the eluting agents and fibrin contaminants. The activator is eluted from the column with arginine, lysine or ε-amino-caproic acid in yields of about 60%. Rijken et al., "Purification and Characterization of the Plasminogen Activator Secreted by Human Melanoma Cells in Culture," J. Biol. Chem., Vol. 256, pp. 7035-41 (1981), describes a purification process using three chromatography steps on zinc chelate-agarose, concanavalin A-agarose and Sephadex G-150 columns, also including dialysis and centrifugation steps, and obtaining a yield of 46%. Similarly, Booyse et al., "Isolation and Characterization of a Urokinase-type Plasminogen Activator ($M_r$ = 55,000) from Cultured Human Endothedial Cells Indistinguishable from Urinary Urokinase," J. Biol. Chem., Vol. 259, pp. 7198-205 (1984), discloses purification from serum-free conditioned medium in the presence of Triton X-100 by p-aminobenzamidine-agarose affinity chromotography, followed by immunoadsorption chromotography on affinity purified specific anti-urokinase IgG-Sepharose CL-4B for yields of about 47%.

## SUMMARY OF THE INVENTION

In sharp contrast to the previously disclosed methods for purifying t-PA, the process of the present invention is simple and efficient. It essentially comprises the steps of contacting conditioned cell culture medium or other t-PA source medium with pretreated fibrin powder or fibrin surface and then eluting the t-PA which becomes bound to the fibrin. Following elution of t-PA from the fibrin, excess t-PA elution buffer may be removed from the eluted t-PA by dialysis or other convenient procedures. t-PA yields of at least 70-75% or higher can been achieved using the process described herein. Moreover, the ultimate t-PA product of this process is a highly purified composition produced with a minimum number of steps.

A primary object of this invention is to provide a streamlined and efficient process for purification of t-PA from conditioned cell culture media, tissues or body fluids. This method eliminates the current need for using multiple gel chromatography and/or immunosorption steps to remove contaminating proteins. This invention offers a purification process in which the t-PA is directly isolated from conditioned media or other sources with minimal contamination by protein impurities, producing a highly purified t-PA product.

It is a closely related object of this invention to provide a method for isolating therapeutic grade t-PA which is at once economical and appropriate for the production of large quantities of purified t-PA to meet the anticipated clinical demands for the material.

A further object is to provide a method of pretreating the fibrin powder to be used for the t-PA purification of this invention in such a manner that contamination of the t-PA product with impurities typically associated with commercially available fibrin is virtually eliminated.

It is an additional object to provide a t-PA purification method based on the direct and specific binding of t-PA from conditioned media or other source material onto an inexpensive, readily available adsorbent, eliminating the need for expensive and/or sophisticated chromatographic media and processing steps to isolate the t-PA in a purified form. The result is production of greater quantities of purified t-PA at a very low cost of isolation.

## DESCRIPTION OF THE INVENTION

According to the process of this invention, t-PA (tissue plasminogen activator) is isolated from a source material such as conditioned cell culture media, tissues or body fluids by the following steps: (1) pretreating fibrin by contacting it with t-PA elution buffer to remove elutable impurities from the fibrin, (2) separating the pretreated fibrin from the t-PA elution buffer, (3) washing the pretreated fibrin, (4) contacting the washed, pretreated fibrin with a source material containing tissue plasminogen activator, (5) separating fibrin-bound t-PA from the source material, (6) washing the fibrin-bound t-PA, and (7) eluting t-PA from the fibrin with t-PA elution buffer. Salts and excess elution buffer may be removed by dialysis or other convenient means. This procedure yields highly purified t-PA.

The t-PA can be isolated from conditioned cell culture media, from various tissues (such as uterine tissue), or from body fluids (such as blood plasma or urine). The terms "source material" and "medium" are used interchangeably herein to refer to a liquid source of t-PA used in the present purification method. Cells which secrete t-PA into culture medium include, but are not limited to, human melanoma cell lines and umbilical vein endothelial cells, The culture medium may be either serum-containing or serum free. t-PA also has been isolated from a variety of other normal and transformed mammalian cells in culture, for example, bovine aortic endothelial cells, delipidized porcine heart tissue, and the like.

It is one of the key elements of the process of this invention that specially pretreated fibrin or fibrin powder is used for binding the t-PA. Pretreated fibrin facilitates the isolation of t-PA from the t-PA source material in a highly purified state. Fibrin powder is available commercially, for example, from Sigma Chemical Co. Fibrin powder or immobilized fibrin may be pretreated and used for t-PA isolation according to the procedures described herein. Throughout this description, "fibrin" or "fibrin powder" will be understood to refer also to a surface prepared with precipitated or immobilized fibrin or to particulate fibrin.

If immobilized fibrin is to be used for t-PA isolation, it may be pre-treated as described either before or after immobilization. Fibrin may, for example, be immobilized or precipitated onto a solid or particulate material for use in a continuous purification process. Materials onto which fibrin may be immobilized or precipitated for use in this process include hollow fibers, amorphous silica, diatomaceous earth, and the like.

The fibrin pretreatment of this invention removes elutable impurities which would otherwise contaminate the t-PA product by co-eluting with the t-PA upon contact with t-PA elution buffer. These contaminants, essentially consisting of various proteins, would require complex purification procedures for their removal. By purifying the fibrin before it is used to isolate the t-PA, the t-PA purification itself is reduced to a single major step. The fibrin pretreatment is easily accomplished. By thoroughly washing the fibrin with the t-PA elution buffer, undesirable proteins, etc. are removed which otherwise would co-elute along with t-PA. Potassium thiocyanate ("KSCN") is the preferred t-PA elution buffer.

Any convenient washing procedure may be used to thoroughly wash the fibrin. For example, fibrin powder may be mixed batch-wise with the t-PA elution buffer, or a continuous process may be employed in which the fibrin or fibrin-covered support is placed in a column or other packed or suspended bed configuration for contact with the t-PA elution buffer. In a continuous process, the fibrin bed may be contacted sequentially with t-PA elution buffer to purify the fibrin, then with the t-PA source medium and finally again with t-PA elution buffer to remove the t-PA which becomes bound to the fibrin.

Thorough washing of the fibrin in the pretreatment step is required. For example, in a batch process, the fibrin may be washed several times, preferably at least about five times, with 1.5 M KSCN solution.

Where KSCN is the t-PA elution buffer, the solution concentration should be at least 1.0 M, preferably at least 1.5 M, up to about 3.0 M. The fibrin should be washed to substantially reduce or eliminate contaminating proteins present in the wash solution as detected by HPLC or direct ultraviolet absorbance readings.

This thorough washing step purifies the fibrin from the low molecular weigh contaminants with which it is associated as commercially available. It is not believed that any prior process has yielded a fibrin composition free from elutable low molecular weight impurities. This purified form of fibrin, which is necessary for use in the t-PA purification process of this invention, is therefore considered to be a novel composition of matter.

The pretreated fibrin and the conditioned medium or other t-PA source material are contacted by any convenient means which will facilitate adequate interface of the t-PA molecules and the pretreated fibrin in order to ensure high levels of TPA-fibrin binding. Batch, semicontinuous or continuous process operations may be employed and adjustments necessitated by specific process configurations will be within the knowledge of persons of ordinary skill in the art. This step preferably is conducted at about 4.0° C, although temperatures up to room temperature, or higher, will be suitable, provided that the conditions are not such as to cause enzyme denaturation. Gentle agitation or stirring is preferred.

Once sufficient contact has been maintained to allow the t-PA to become bound to the pretreated fibrin, the fibrin is separated from the conditioned medium or other source material. In a batch process, separation may be by centrifugation or filtration, for example. The fibrin, now comprising bound t-PA, is washed with buffer. A suitable buffer is sodium phosphate buffer (pH 5-9). The preferred buffer is PBS with .01% Tween 80 (TM) (ICI United States, Inc.). The buffer used in this step is not the "t-PA elution buffer" described and referred to elsewhere in this description.

The t-PA is then eluted from the fibrin with t-PA elution buffer. For this purpose, 1.5 M KSCN may be used and is the preferred t-PA elution buffer. Elution is conducted until no further t-PA is eluted. It has been found that very small volumes of elution buffer are not sufficient for eluting the t-PA. It is therefore preferred that at least about 1.0 ml elution buffer be used for every 5.0 μgm t-PA to be eluted. The t-PA elution buffer and salts then are removed from the purified t-PA by dialysis against PBS with .01% Tween 80 (TM) with a 1000 MW cellulose acetate membrane. The dialysis step, which produces highly purified t-PA, is necessary before the t-PA can be used for injection into human patients. A polishing step of ultrafiltration removes any solubilized fibrin which may be present.

The t-PA isolated by the process described above is highly purified. This t-PA is substantially free of contaminating proteins and other contaminating materials. Conventional purification methods yield t-PA which is contaminated with several other proteins. Even the pure t-PA presently available for reference standard use contains contaminating proteins. By contrast, t-PA isolated by the method of this invention is characterized by a single major protein band on SDS-PAGE analysis, corresponding to authentic t-PA of approximately 90% purity.

The examples which follow are given for illustrative purposes and are not meant to limit the invention described herein. The following abbreviations have been used throughout in describing the invention.

° C - degree(s) Centigrade
cm³ - cubic centimeter(s)
gm - gram(s)
HPLC - high performance liquid chromatography
KSCN - potassium thiocyanate
M - molar
min - minute(s)
ml - milliliter(s)
MW - molecular weight
PBS - phosphate buffered saline
% - percent
SDS - sodium dodecyl sulfate
SDS-PAGE - SDS polyacrylamide gel electrophoresis
t-PA - tissue plasminogen activator
μgm - microgram(s)

EXAMPLE I

4

Fibrin Pretreatment -

One gram of fibrin powder (Sigma Chemical Co.) was stirred with 40.0 ml of 1.5M KSCN solution for one hour to remove undesirable elutable proteins. This washing was repeated for a total of five washes. The treated fibrin powder was separated from the KSCN by centrifugation and was washed with IM PBS (pH 7.0).

t-PA Purification -

A 50.0 ml volume of conditioned medium containing 15.6 μgm t-PA was stirred for two hours at room temperature with one gram of fibrin powder that had been pretreated as described above. Next, the fibrin powder (now containing bound t-PA) was separated from the spent medium by centrifugation and was washed five times with 50.0 ml volumes of saline solution. The washed fibrin powder was stirred with 10.0 ml of 1.5M KSCN solution for one hour at room temperature to elute the bound t-PA. The fibrin powder was separated from the KSCN eluate by centrifugation. The eluate was ultrafiltered to remove salts.

The eluate concentrate was found to contain 11.07 μgm of enzymatically active t-PA by a standard lysis zone assay using a fibrin agarose plate. In this assay, plates containing precipitated fibrin in agar are lysed by enzymatically active t-PA; the zone size corresponds to the quantity of t-PA. The level of observed enzymatic activity in this Example represents a 71% recovery of the total t-PA in the starting medium. The isolated t-PA was subjected to SDS-PAGE gel analysis and found to be of high purity.

## EXAMPLE II

Fibrin Pretreatment -

Two grams of fibrin (Sigma Chemical Co.) were washed five times with 50.0 ml of 1.5M KSCN to remove contaminating proteins. After the fifth KSCN wash, no contaminating proteins were detected by HPLC in the wash solution. The treated fibrin powder was separated from the KSCN by centrifugation and then was washed with 1M PBS (pH 7.0).

t-PA Purification -

A 200 ml volume of conditioned media containing 833 μgm t-PA was stirred with 2.0 gm of the pretreated fibrin for two hours at room temperature. The fibrin-TPA complex was separated and the t-PA eluted as in Example I.

Residual t-PA activity in the treated conditioned medium was estimated by fibrin clot lysis assay, which indicated that 625.0 μgm t-PA (about 75% of the t-PA in the conditioned media) had been bound by the pretreated fibrin. After elution, as described in Example I, with 2M KSCN, it was estimated by fibrin clot lysis assay that 548.0 μgm t-PA had been eluted, in 45.0 ml total volume of eluate. SDS-PAGE analysis showed a single major protein band (about 62,000 MW) corresponding to authentic t-PA of approximately 90% purity.

Controls were run in this example, using unwashed or partially washed fibrin in place of the pretreated fibrin described above. "Partial washing" is defined as washing one, two, three or four times with KSCN as described above. In all controls, complete binding of the t-PA from the conditioned media was observed. However, the eluted t-PA in the unwashed and partially washed fibrin controls was contaminated with at least three nonspecifically bound or endogenous fibrin proteins, as shown by SDS-PAGE analysis.

## EXAMPLE III

Scale up of the t-PA purification process described above was accomplished using precipitated fibrin on hollow fibers. Fibrinogen (35 gm) was immobilized by passive adsorption on large volume (4.0 ml) hollow fibers. After washing, 5.0 μgm thrombin in a carbonate buffer containing 1.0 mM CaCl₂ were circulated through the cartridge for three hours.

A 400.0 ml volume of conditioned media containing 500 μgm t-PA was circulated through the prepared hollow fiber cartridge overnight at 4.0°C at a flow rate of 3.0 ml/min for maximum binding of t-PA to the precipitated fibrin. Essentially all of the t-PA in the medium bound to the fibrin. The t-PA was then eluted by circulating eluting buffer (1.5M KSCN) through the cartridge after first washing with PBS.

## EXAMPLE IV

Purity comparisons were made between commercially available t-PA and the t-PA purified by the method of this invention. The experimental t-PA sample was purified as in Example III. Commercial t-PA was obtained from CES Ltd. Purity comparisons were conducted by SDS-PAGE analysis. It should be noted, however, that all commercial t-PA is sold in combination with BSA (bovine serum albumin) or HSA (human serum albumin), which are used as stabilizers, and which make purity comparisons difficult. The experimental t-PA purified by the method of this invention yielded a single protein band. The commercial t-PA yielded multiple protein bands.

## EXAMPLE V

Fibrin cartridges prepared as in Example III, above, were recycled for purification of two additional volumes of TPA-containing conditioned medium. Table 2 shows the volumes and concentrations of t-PA in the conditioned medium, as well as the results of the purification cycles. It is apparent from these data that the t-PA purification process of this invention is adaptable to continuous processing modes.

TABLE 2

| Cycle | Medium Volume | μgm t-PA Offered | %TPA Bound | μgm t-PA Eluted | %TPA Eluted |
|-------|---------------|------------------|------------|-----------------|-------------|
| 1 | 400 ml | 1000 | 100 | 832 | 83 |
| 2 | 221 ml | 554 | 100 | 525 | 94 |
| 3 | 298 ml | 747 | 86 | 712 | 95 |

The principles, preferred embodiments and modes of operation of the present invention have been described in the foregoing specification. The invention which is intended to be protected herein, however, is not to be construed as limited to the particular forms disclosed, since these are to be regarded as illustrative rather than restrictive. Variations and changes may be made by those skilled in the art without departing from the spirit of the invention.

## Claims

1. A method for isolating t-PA from a source material by:
   (a) pretreating fibrin by contacting it with t-PA elution buffer to remove elutable impurities from the fibrin,
   (b) separating the pretreated fibrin from the t-PA elution buffer,
   (c) washing the pretreated fibrin,
   (d) contacting the washed, pretreated fibrin with a source material containing t-PA,
   (e) separating fibrin-bound t-PA from said source material,
   (f) washing fibrin-bound t-PA, and
   (g) eluting t-PA from the fibrin with t-PA elution buffer.

2. The method of claim 1 in which said fibrin is fibrin powder or is fibrin which has been precipitated onto a surface, whereby said surface is a hollow fiber membrane or is inert particulate matter.

6

3. The method of claims 1 or 2 in which the t-PA elution buffer used in step (a) is potassium thiocyanate.

4. The method of claims 1 to 3 in which said fibrin is pretreated by thoroughly washing it with t-PA elution buffer to substantially reduce or eliminate the presence of contaminating proteins in the wash solution.

5. The method of claims 1 to 4 which includes subjecting the t-PA eluted from the fibrin in step (g) to dialysis treatment.

6. Highly purified t-PA which is free from contaminating proteins.

7. Highly purified fibrin, characterized by being substantially free of impurities capable of elution with t-PA elution buffer.

8. The highly purified fibrin of claim 8, prepared by thoroughly washing fibrin with t-PA elution buffer.

9. The highly purified fibrin of claim 9, prepared by thoroughly washing with potassium thiocyanate.

10. The highly purified fibrin of claim 9 which is washed with phosphate buffered saline.